Europäisches Patentamt

European Patent Office

Office européen des brevets

(11) Publication number: **0 241 618**
**A2**

## (12) EUROPEAN PATENT APPLICATION

(21) Application number: 86309918.0

(51) Int. Cl.⁴: **A61K 31/165**

(22) Date of filing: 18.12.86

| | |
|---|---|
| The title of the invention has been amended (Guidelines for Examination in the EPO, A-III, 7.3). | (71) Applicant: **ELI LILLY AND COMPANY Lilly Corporate Center Indianapolis Indiana 46285(US)** |
| (30) Priority: 20.12.85 US 811918 | (72) Inventor: **Boder, George Bernard 1008 Maple Turn Road Martinsville Indiana 46151(US)** |
| (43) Date of publication of application: **21.10.87 Bulletin 87/43** | Inventor: **Grindey,Gerald Burr 5223 East 77th Street Indianapolis Indiana 46250(US)** |
| (84) Designated Contracting States: **AT BE CH DE FR GB IT LI LU NL SE** | (74) Representative: **Tapping, Kenneth George et al Erl Wood Manor Windlesham Surrey, GU20 6PH(GB)** |

(54) Phenyl ethanol amine derivative for use as an oncolytic medicament.

(57) Intravenous administration of a pharmaceutically acceptable acid addition salt of a $\beta$-phenethanolamine produces good antitumor activity.

EP 0 241 618 A2

## ONCOLYTIC METHOD

The chemistry and pharmacology of β-phenethanolamines has been extensively investigated. Numerous β-phenethanolamines are known to possess cardiovascular activities and are useful as antihypertensive agents, inotropic agents and the like. See U.S. Patent Nos. 4,086,272 and 4,217,305. Certain β-phenethanolamines have been found effective as antiobesity agents; U.S. Patent Nos. 4,391,826 and 4,338,333. Other β-phenethanolamines are said to be useful in promoting growth in domesticated animals. See EP 26,296 and EP 49,728. Mills et al., in European Patent No. 7206 issued February 1983, describe certain β-phenethanolamines which are said to potentiate the activity of known oncolytic agents such as 6-mercaptopurine, 5-fluorouracil, cytoxan and the like. Mills et al. specifically teach that the disclosed β-phenethanolamines are pure potentiators of oncolytic agents and do not possess antineoplastic activity themselves.

We have now discovered that a β-phenethanolamine of the Mills et al. type possesses good antineoplastic activity by itself when administered to an animal by the intravenous route.

This invention concerns a method for treating neoplastic diseases comprising administering via the intravenous route a β-phenethanolamine to an animal in need of treatment. More particularly, this invention provides a method for treating tumors in animals such as humans comprising administering by the intravenous route an antitumor effective amount of R-N-(2-phenyl-2-hydroxyethyl)-1,1-dimethyl-3-(4-aminocarbonylphenyl)-propylamine as a pharmaceutically acceptable acid addition salt.

A further aspect of the invention is an intravenous formulation for use in the therapy of malignant tumors in humans comprising a pharmaceutically acceptable acid addition salt of R-N-(2-phenyl-2-hydroxyethyl)-1,1-dimethyl-3-(4-aminocarbonylphenyl)propylamine and a diluent or carrier.

The present method of treatment requires the β-phenethanolamine salt be administered by the intravenous route. The compound is accordingly formulated with commonly employed excipients and diluents to produce physiologically acceptable solutions or suspensions suitable for intravenous injection or infusion. The pharmaceutically acceptable acid addition salts have good solubility in common aqueous diluents such as water, dilute saline and dilute glucose solutions.

Typical pharmaceutically acceptable acid addition salts include those made by reacting the free amine with an inorganic acid such as hydrochloric, sulfuric, perchloric, nitric or phosphoric acid, or an organic acid such as acetic acid, succinic acid, butyric acid, maleic acid lactic acid, oxalic acid, p-toluene-sulfonic acid, or the like. A preferred salt for use in the present method is that made with hydrochloric acid, i.e., the hydrochloride.

The β-phenethanolamine salts required for the invention are suspended or dissolved in common diluents and excipients for use in the present method. Typical excipients and diluents commonly employed include isotonic saline, 5% aqueous dextrose, water and the like. Formulations having suitable stability over prolonged periods (e.g., shelf life) generally are in the form of dry powders. The β-phenethanolamine defined above can be maintained in a vial as a dry crystalline powder, or more preferably a combination of the β-phenethanolamine with an excipient such as mannitol, sucrose, starch, dextrose, or the like is dissolved in water and lyophilized to a stable dry powder. The dry powder is readily reconstituted prior to use by addition of a suitable diluent such as water, phosphate buffered saline, and the like.

The oncolytic method provided by this invention is practiced by administering the compound defined herein by the intravenous route to an animal in need of treatment. All that is required to practice the invention method is that an effective amount of the β-phenethanolamine as a pharmaceutically acceptable acid addition salt be administered I.V. to the animal. The animal to be treated will be a human in a preferred embodiment. While the precise amount of β-phenethanolamine to be administered will be determined by the particular tumor to be treated, the severity of the disease, the general condition of the subject to be treated, and related factors that only the attending physician can determine, the typical effective amount will be from about 1 to about 200 mg/kg, and more normally from about 20 to about 100 mg/kg. Such doses will be given from one to two times daily, or more often as required to effectively treat the afflicted subject. The compound can be administered by injection or infusion into the intravenous system of an animal, and is preferably administered rapidly as an I.V. injection.

The compounds to be employed in the method of this invention can be prepared by the general procedures described in Mills et al. EP 7206. A preferred synthesis of R-N-(2-phenyl-2-hydroxyethyl)-1,1-dimethyl-3-(4-aminocarbonylphenyl)propylamine and its hydrochloride was carried out as follows.

## Example 1

A solution of 2.3 kg of 1,1-dimethyl-3-(4-aminocarbonylphenyl)propylamine in 4.0 liters of dimethyl sulfoxide and 1.11 liters of hexamethyldisilazane was heated at 75°C for twenty-four hours. To the hot solution were added portionwise 1.42 kg of R-styrene oxide. The reaction mixture was heated for an additional twenty-four hours at 75°C. The mixture was cooled to 10°C and diluted by addition of 7.5 liters of water. The aqueous mixture was acidified to pH 1.0 by addition of 1.0 liter of concentrated hydrochloric acid. After stirring the aqueous acid solution for one hour at 25°C, the solution was washed twice with 2.0 liter portions of dichloromethane. The acidic solution was layered with 4.0 liters of tetrahydrofuran and the pH was adjusted to 13 by slow addition of 50% aqueous sodium hydroxide. The organic layer was separated and the aqueous alkaline solution was further extracted with 6.0 liters of fresh tetrahydrofuran. The organic extracts were combined, concentrated to about 5.0 liters by evaporation under reduced pressure, diluted by addition of 10.0 liters of methanol, and again concentrated to a volume of about 6.0 liters. The concentrated solution was heated to 65°C and then diluted by addition of 10.0 liters of water. The mixture was cooled to 25°C for twelve hours, and then to 15°C. The precipitate was collected by filtration, washed with 5.0 liters of water and air dried to provide 2026 g (63.1% yield) of R-N-(2-phenyl-2-hydroxyethyl)-1,1-dimethyl-3-(4-aminocarbonylphenyl)propylamine.

M.P. 167-169°C. $[\alpha]_{MeOH}^{589}$ = -28.28°

Elemental Analysis Calc. for $C_{20}H_{26}N_2O_2$

Theory: C, 73.59; H, 8.03; N, 8.58.

Found: C, 73.34; H, 7.84; N, 8.32.

The product from above was added portion-wise to 10.8 liters of ethanol. Anhydrous hydrogen chloride (242 g) was bubbled into the reaction mixture while the temperature was maintained at 20-42°C. The reaction solution was stirred for twelve hours at 25°C and the crystalline precipitate was collected by filtration, washed with fresh alcohol and dried at 45°C to provide 1908 g of R-N-(2-phenyl-2-hydroxyethyl)-1,1-dimethyl-3-(4-aminocarbonylphenyl)propylamine hydrochloride.

M.P. 194-195°C. $[\alpha]_{MeOH}^{589}$ = -37.69° $[\alpha]_{MeOH}^{365}$ = -119.44°

Elemental Analysis Calc. for $C_{20}H_{27}N_2O_2Cl$

Theory: C, 66.19; H, 7.50; N, 7.72; Cl, 9.77.

Found : C, 66.08; H, 7.44; N, 7.65; Cl, 10.02.

Exemplary formulations of the above-described compound suitable for intravenous administration according to this invention include the following:

## Example 2

R-N-(2-phenyl-2-hydroxyethyl)-1,1-dimethyl-3-(4-aminocarbonylphenyl)propylamine hydrochloride (2.0 grams) is dissolved in 10 ml of isotonic saline. The solution is administered I.V. dropwise over ten minutes.

## Example 3

R-N-(2-phenyl-2-hydroxyethyl)-1,1-dimethyl-3-(4-aminocarbonylphenyl)propylamine hydrochloride (1.0 gram) is dissolved in 1.0 ml of 5% aqueous dextrose solution. The solution is administered IV rapidly to a subject suffering from a solid tumor.

## Example 4

R-N-(2-phenyl-2-hydroxyethyl)-1,1-dimethyl-3-(4-aminocarbonylphenyl)propylamine hydrochloride (250 mg) is dissolved in 10 ml of water containing 250 mg of mannitol. The solution is lyophilized to provide 500 mg of white powder in a vial. The contents of the vial are reconstituted by addition of 9.7 ml of water for injection by the intravenous route.

The β-phenethanolamine employed in the invention method is effective against a wide range of tumors, including adenocarcinomas, mammary carcinomas, colon carcinomas, ovarian tumors, lung carcinomas, myelomas and the like.

The potent oncolytic activity of the compound to be employed in the present method of treatment has been established in standard tests designed to evaluate antitumor activity. Cell biology studies have demonstrated the compound, i.e., R-N-(2-phenyl-2-hydroxyethyl)-1,1-dimethyl-3-(4-aminocarbonylphenyl)-propylamine hydrochloride, is an antimitotic agent that acts by a mechanism other than the standard antitubulin compounds such as the vinca alkaloids. The subject compound appears to affect only the spindle microtubules, with no apparent effect on cytoplasmic microtubules. The effect on spindle; microtubules appears to reflect a specific effect on the microtubule organizing center of the spindle, i.e., the centrosomes. These studies have been documented by immunofluorescent light microscopy, transmission electron microscopy and flow cytometric techniques. Additional studies have indicated the compound causes an S-phase accumulation of Chinese hamster ovary cells grown in low calcium medium. In cell culture, the compound has antimitotic activity at 2.5 μg/ml and affects mitochondrial function at 0.01 μg/ml as measured by alterations in Rhodamine 123 binding.

The compound of the above example has been extensively evaluated for oncolytic activity in in vivo laboratory studies. In a typical experiment, laboratory mice were infected by subcutaneous implantation of a tumor tissue in the axillary region of the body. The implantations were accomplished by means of a trocar. Initial tumor size in each animal generally was about 2 square millimeters. Control animals received saline. Tumors were measured using vernier calipers (length and width in mm) following a specified period of treatment. The length and width measurements were converted to tumor weight by the formula:

The percent inhibition of tumor growth was then calculated for each dose level at which the compound was evaluated. Typical tumor systems implanted and against which the compound was evaluated include the CA-755 strain of adenocarcinoma, the M-5 ovarian carcinoma, the 6C3HED lymphosarcoma, the X-5563 plasma cell myeloma, the Lewis lung carcinoma, colon carcinoma-26, and C3H mammary adenocarcinoma.

Tables I below presents the results of in vivo studies carried out as described above. Each test group consisted of ten mice. R-N-(2-phenyl-2-hydroxyethyl)-1,1-dimethyl-3-(4-aminocarbonylphenyl)propylamine hydrochloride was dissolved in 5% aqueous saline and administered twice daily for five days as a 0.2 ml intravenous injection. Treatment was initiated on the day following inoculation of the test animals.

## Table I

Dose (mg/kg)   % Inhibition

CA-755 Adenocarcinoma

| | Dose (mg/kg) | % Inhibition |
|---|---|---|
| Control | 0 | 0 |
| Compound | 80 | 95 |
| | 40 | 92 |
| | 20 | 95 |
| | 10 | 79 |

6C3HED Lymphosarcoma

| | | |
|---|---|---|
| Control | 0 | 0 |
| Compound | 80 | 23 |
| | 40 | 9 |
| | 20 | 11 |

X-5563 Plasma Cell Myeloma

| | | |
|---|---|---|
| Control | 0 | 0 |
| Compound | 80 | 65 |
| | 40 | 21 |
| | 20 | 29 |

## Table I (continued)

|  | Dose (mg/kg) | % Inhibition |
|---|---|---|
| **Lewis Lung Carcinoma** | | |
| Control | 0 | 0 |
| Compound | 80 | 81 |
|  | 40 | 68 |
|  | 20 | 35 |
| **C3H Mammary Adenocarcinoma** | | |
| Control | 0 | 0 |
| Compound | 80 | 96 |
|  | 40 | 95 |
|  | 20 | 57 |

Table II presents the results of studies carried out substantially as described above, except the treatment regimens were delayed for a short period following inoculation to permit the tumor to become established and start growing. Treatment consisted of I.V. injection of 0.2 ml of the appropriate amount of R-N-(2-phenyl-2-hydroxyethyl)-1,1-dimethyl-3-(4-aminocarbonylphenyl)propylamine hydrochloride in 5% aqueous saline twice daily for five days. Tumors were then measured as described above and compared with untreated controls.

## Table II

### Colon Carcinoma-26

| | Days delay following innoculation | Dose (mg/ml) | %Inhibition | Deaths/total mice in group |
|---|---|---|---|---|
| Control | 3 | 0 | 0 | 0/10 |
| Compound | | 80 | 64 | 4/9 |
| | | 40 | 61 | 1/10 |
| | | 20 | 30 | 0/10 |

### M-5 Ovarian Carcinoma

| | Days delay following innoculation | Dose (mg/ml) | %Inhibition | Deaths/total mice in group |
|---|---|---|---|---|
| Control | 5 | 0 | 0 | 0/10 |
| Compound | | 80 | 81 | 0/10 |
| | | 40 | 61 | 0/10 |
| | | 20 | 40 | 0/10 |

## Claims

1. The use of a pharmaceutically-acceptable acid addition salt of R-N-(2-phenyl-2-hydroxyethyl)-1,1-dimethyl-3-(4-aminocarbonylphenyl)propylamine for the manufacture of an intravenous formulation for the treatment of tumors in animals.

2. The use claimed in Claim 1, wherein the animal to be treated is a human.

3. The use claimed in Claim 1 or 2, wherein the salt is R-N-(2-phenyl-2-hydroxyethyl)-1,1-dimethyl-3-(4-aminocarbonylphenyl)propylamine hydrochloride.

4. The use claimed in any one of claims 1 to 3 wherein the tumor to be treated is a solid tumor.